# EUROPÄISCHE PATENTANMELDUNG

(11) **EP 3 960 843 A1**
(43) Veröffentlichungstag der Anmeldung: **02.03.2022**
(21) Anmeldenummer: 21193955.8
(22) Anmeldetag: 31.08.2021
(51) Int. Cl.: C12M 1/107

(54) **GÄRBEHÄLTERANORDNUNG MIT DIREKTFILTRATION**

(30) Priorität: 01.09.2020 DE 202020105026 U
(71) Anmelder: PlanET Biogas Group GmbH, 48691 Vreden (DE)
(72) Erfinder: FORTMEYER, Jörgen, 48691 Vreden (DE); MEYER ZU STROHE, Jörg Ernst, 46342 Velen (DE)
(74) Vertreter: Habbel, Ludwig

(57) **Zusammenfassung**

Bei einer Gärbehälteranordnung einer Biogasanlage, mit einer Rohrleitung, die einen als Zuleitung (8) bezeichneten, von einem Gärbehälter (1) zu einem Einlass (9) eines Förderfilters (5) führenden Abschnitt, wobei als Förderfilter (5) eine Filtrationseinrichtung bezeichnet ist, die ein oder mehrere bewegliche Elemente aufweist, die auf das zu filternde Medium eine aktive Förderwirkung ausüben, und einen als Rückleitung bezeichneten, von einem Feststoffauslass (7) des Förderfilters (5) zum Gärbehälter (1) führenden Abschnitt aufweist, wobei die Zuleitung (8) dazu bestimmt ist, Substrat aus dem Gärbehälter (1) zu dem Förderfilter (5) zu führen, und wobei die Rückleitung dazu bestimmt ist, eine im Förderfilter (5) aus dem Substrat abgeschiedene feste Phase in den Gärbehälter (1) zurückzuführen, schlägt die Erfindung vor, dass die Zuleitung (8) vom Gärbehälter (1) abwärts zum Einlass (9) des Förderfilters (5) verläuft, und dass der Förderfilter (5) an den Gärbehälter (1) in der Art angeflanscht ist, dass die feste Phase mittels einer Förderwirkung des Förderfilters (5) in den Gärbehälter (1) förderbar ist.

## Beschreibung

Die Erfindung betrifft eine Gärbehälteranordnung nach dem Oberbegriff des Anspruchs 1. Als Gärbehälter einer Biogasanlage kommen beispielsweise Fermenter, Nachgärer, Gärproduktlager oder Hydrolysebehälter in Frage.

Da in jüngerer Vergangenheit das Augenmerk vermehrt auf Reststoffe und Wirtschaftsdünger in Form von Gülle gelegt wird, kann das Substrat in einem Gärbehälter wie z. B. einem Fermenter eine unerwünscht geringe Energie- und Faserstoffdichte aufweisen. Dies führt zum einen dazu, dass große Mengen an Substrat benötigt werden, um eine bestimmte Biogasausbeute zu erreichen. Da dies aus wirtschaftlichen Gründen bei möglichst kleinen Fermenter-Baugrößen erfolgen soll, wird dementsprechend eine kurze Verweilzeit des Substrats in dem betreffenden Gärbehälter eingestellt.

Dies führt zum einen dazu, dass die wenigen noch vorhandenen Faserstoffe schneller ausgetragen werden, was den Biogasertrag pro Tonne Frischmasse reduziert, da die Verweilzeit nicht ausreicht, um die Faserstoffe aufzuschließen, sondern der Großteil des Ertrages nur aus den schnell verfügbaren Stoffen erzielt wird. Gleichzeit tritt jedoch das Problem auf, dass kaum Material vorhanden ist, auf dem sich die Mikroorganismen ansiedeln können. Die Mikroorganismen, die sich ansiedeln, werden aufgrund der vergleichsweise kurzen Verweilzeit schneller wieder aus dem Gärbehälter ausgespült, sodass der Aufbau eines biologisch stabilen Prozesses erschwert wird.

Die DE 10 2010 033 442 A1 beschreibt ein Verfahren zum Betreiben einer Biogasanlage unter Verwendung eines gattungsgemäßen Gärbehälters, wobei aus dem Gärbehälter der Biogasanlage Substrat abgezogen wird, dass Substrat in eine feste und eine flüssige Phase getrennt wird, die flüssige Phase ausgeschleust wird und die feste Phase in den Gärbehälter zurückgeführt wird. Auf diese Weise kann der Anteil der energiereichen festen Phaserstrukturen im Gärbehälter vergrößert werden, um die Biogasanlage leistungsfähiger zu machen und den Ertrag an Biogas zu vergrößern.

Als Weiterentwicklung dieses bekannten Verfahrens beschreibt die DE 10 2011 117 990 A1, dass die Leistungsfähigkeit des Verfahrens über eine Druckregelung verbessert werden kann, um energiereiche Substratbestandteile von energieärmeren Bestandteilen zu trennen und somit die Biogasausbeute zu verbessern.

Die zur Durchführung der bekannten Verfahren verwendeten Anlagen verursachen je nachdem, wie aufwendig ihre apparative Ausgestaltung ist, dementsprechend hohe Investitions- und Unterhaltskosten. Zudem können sie je nachdem, wie lang die Rohrleitungen zwischen den einzelnen Komponenten der Anlage sind, zu Temperaturverlusten des Substrats führen, was die biologische Umsetzung des Substrats negativ beeinflusst und somit die Stabilität der biologischen Prozesse und die Leistungsfähigkeit der Biogasanlage beeinträchtigt.

Der Erfindung liegt die Aufgabe zugrunde, eine gattungsgemäße Gärbehälteranordnung einer Biogasanlage dahingehend zu verbessern, dass eine Filtration des Substrats mit einem möglichst geringen apparativen Aufwand und dementsprechend wirtschaftlich ermöglicht wird, negative Einflüsse auf das Substrat durch Temperaturverluste möglichst vermieden werden und ein biologisch möglichst stabiler Betrieb des Gärbehälters ermöglicht wird.

Diese Aufgabe wird durch eine Gärbehälteranordnung mit den Merkmalen des Anspruchs 1 gelöst. Vorteilhafte Ausgestaltungen sind in den Unteransprüchen beschrieben.

Die Erfindung schlägt mit anderen Worten vor, einen Teilstrom des Gärbehälterinhalts während des Fermentationsprozesses durch ein Direktfiltrationssystem zu führen und unter Ausschluss von Sauerstoff einzudicken. Als Direktfiltration wird im Rahmen der vorliegenden Erfindung verstanden, dass das Substrat über möglichst kurze Wege zu einem Förderfilter und wieder zurück in den Gärbehälter geführt wird. Dies wirkt sich in zweifacher Hinsicht vorteilhaft aus: erstens ist die Gesamtlänge der als Zu- und Rückleitung dienenden Rohrleitungen möglichst kurz bemessen, was bereits für sich genommen die Investitionskosten zu minimieren hilft. Zweitens werden Wärmeverluste minimiert, da das Substrat typischerweise ein Temperaturniveau oberhalb der Umgebungstemperatur aufweist. Ebensowenig ist eine aufwändige Dämmung der Zu- und Rückleitung erforderlich. Und schließlich werden Rohrleitungs- bzw. Strömungswiderstände minimiert, so dass mittels der Direktfiltration der Gärbehälter energetisch besonders effizient betrieben werden kann.

Als Förderfilter wird im Rahmen der vorliegenden Erfindung eine Filtrationseinrichtung bezeichnet, die nicht nur die mit Durchtrittsöffnungen versehene Filterfläche aufweist und möglicherweise passiv durchströmt wird, beispielsweise mit Hilfe einer zusätzlich vorhandenen externen Pumpe, sondern die vielmehr selbst eines oder mehrere bewegliche Elemente aufweist, die auf das zu filternde Medium, beispielsweise das Substrat einer Biogasanlage, eine aktive Förderwirkung ausüben, also dieses Medium auch ohne Anwesenheit einer externen Pumpe in Strömung versetzen. Aus der Praxis sind derartige Förderfilter in unterschiedlichen konstruktiven Ausgestaltungen bekannt, beispielsweise als Separator, Dekanter, Wendelfilter oder dergleichen.

Beispielsweise ist es bekannt, auch bei einer gattungsgemäßen Gärbehälteranordnung als Filtrationseinrichtung einen Förderfilter zu verwenden, beispielsweise in Form eines Separators. Die gattungsgemäße Anordnung, bestehend aus dem Gärbehälter einerseits und dem Förderfilter andererseits sowie aus den dazwischen verlaufenden Rohrleitungen und Pumpen, macht von der Förderwirkung des Förderfilter keinen nennenswerten Gebrauch, vielmehr beschränkt sich der Nutzen des Förderfilters bei den bekannten Gärbehälteranordnungen auf die Filtrationswirkung des Förderfilters.

Die erfindungsgemäß vorgesehene Direktfiltration wird in der Art ermöglicht, dass der Förderfilter an den Gärbehälter angeflanscht ist, entweder unmittelbar oder mit einem so kurzen als Rückleitung dienenden Rohrleitungsabschnitt, dass der Förderfilter förderwirksam genutzt wird, indem die im Förderfilter aus dem Substrat abgetrennte feste Phase durch die Förderwirkung des Förderfilters in den Gärbehälter zurück gefördert wird. Um die gewünschte Filtration des Substrats zu bewirken, sind im Förderfilter nämlich Elemente angeordnet, die eine Materialströmung des Substrats bewirken, insbesondere seiner festen Phase. Beispielsweise kann im Inneren des Förderfilters eine um ihre Längsachse drehbare Schnecke angeordnet sein, die als Förder- und Pressschnecke wirkt, und die von Siebblechen umgeben ist, so dass die feste Phase in Längsrichtung der Schnecke gefördert wird und die flüssige Phase durch die Siebbleche radial nach außen hindurchtritt. Die typische Kreislauf- oder Umwälz- oder Förderpumpe, die ansonsten erforderlich ist, um die feste Phase gegen den im Gärbehälter herrschenden Innendruck in den Gärbehälter zu führen, oder um Höhenunterschiede zu überwinden, kann dementsprechend entweder besonders klein dimensioniert werden oder, was als besonders vorteilhafte Ausgestaltung angesehen wird, gänzlich entfallen.

Um das Substrat aus dem Gärbehälter in den Förderfilter zu fördern, ist erfindungsgemäß ebenfalls keine Pumpe erforderlich, denn die Zuleitung, die vom Gärbehälter zum Förderfilter führt, verläuft erfindungsgemäß vom Gärbehälter abwärts zum Einlass des Förderfilters, so dass allein durch die Wirkung der Schwerkraft das Substrat aus dem Gärbehälter in den Förderfilter gelangt.

Durch die Direktfiltration und den damit verbundenen verringerten apparativen Aufwand werden aber nicht nur Investitions- und Betriebskosten vorteilhaft minimiert, sondern auch die Betriebssicherheit der gesamten Anlage verbessert, da durch die Reduzierung der verwendeten Bauteile und Komponenten die Fehler- oder Ausfallwahrscheinlichkeit reduziert wird, und da durch die reduzierte Rohrleitungslänge auch die Anzahl von Rohr-Verbindungsstellen reduziert werden, kann dementsprechend die Wahrscheinlichkeit von Leckagen verringert werden kann.

Abgesehen von der festen Phase fällt im Förderfilter auch eine flüssige Phase an, die beispielsweise zwischengelagert oder abgeleitet werden kann. Dementsprechend ist vorgesehen, dass eine Flüssigkeitsleitung dazu dient, die flüssige Phase aus dem Förderfilter zu führen. In einer Ausgestaltung kann vorgesehen sein, dass der Förderfilter ausreichend hoch oberhalb des Bodenniveaus des Gärbehälters angeordnet ist, so dass die flüssige Phase allein durch die Wirkung der Schwerkraft abfließen kann. Beispielsweise kann der Förderfilter in einem Abstand von 2 m oder höher oberhalb des Bodenniveaus des Gärbehälters angeordnet sein. Alternativ - oder auch ergänzend dazu - kann vorgesehen sein, dass in die Flüssigkeitsleitung eine Förderpumpe eingeschaltet ist, um beispielsweise besonders lange Wegstrecken oder einen Höhenunterschied zu überwinden, oder um die flüssige Phase gegen den Innendruck in einen Tank fördern zu können.

In einer vorteilhaften Ausgestaltung kann die Zuleitung als Rohrbogen ausgestaltet sein. An Stelle einer schräg abwärts verlaufenden Zuleitung sind dementsprechend keine schrägwinkligen Anschlüsse der Zuleitung an den Gärbehälter und an den Förderfilter erforderlich, sondern unter Verwendung von handelsüblichen Normteilen kann die Zuleitung geschaffen und mit handelsüblichen Flanschverbindungen an den Gärbehälter und an den Einlass des Förderfilters angeschlossen werden.

Zwar kann grundsätzlich vorgesehen sein, eine Unterstützungspumpe in den Kreislauf der festen Phase einzubinden, also entweder in die Zuleitung, wo die feste Phase innerhalb des gesamten Substrats vorliegt, oder in die Rückleitung, wo ausschließlich die im Förderfilter abgetrennte feste Phase zum Gärbehälter zurückströmt. Als besonders vorteilhaft ist jedoch eine Ausgestaltung des Gärbehälters vorgesehen, die aufgrund der Direktfiltration auf eine Pumpe innerhalb des Kreislauf der festen Phase völlig verzichtet, so dass weder in der Zuleitung noch in der Rückleitung eine Pumpe angeordnet ist und die Strömung innerhalb dieses Kreislaufs ausschließlich durch die Schwerkraft und durch die Förderwirkung des Förderfilters erzeugt wird. Sowohl die Investitionskosten als auch die Betriebskosten können auf diese Weise besonders niedrig gehalten werden.

Durch die erfindungsgemäße Ausgestaltung des Gärbehälters wird ein Verfahren ermöglicht, das an einer Biogasanlage mit mindestens einem Behälter während des laufenden Vergärungsprozesses die Möglichkeit eröffnet, das Gärsubstrat durch eine Filtrationseinheit zu führen, mit der dann das Substrat in der Art eingedickt werden kann, dass möglichst alle Faserstoffe und Mikroorganismen zurückgehalten werden. Diese werden dann in den Gärbehälter zurückgeführt. Die abgesonderte, annähernd ausgegorene und mikroorganismenreduzierte Flüssigkeit kann für die weitere Verwendung gelagert bzw. abgeleitet werden.

Im Idealfall geschieht der gesamte Abfluss des Gärrestes durch das Filtrationssystem, somit ist gewährleistet, dass sämtliches Material, das den Gärbehälter verlässt, durch den Filter gelangt. Dies erhöht die Verweilzeit der Faserstoffe theoretisch auf unendlich. In der Praxis wird die Verweilzeit jedoch dadurch begrenzt, dass die Faserstoffe durch die Mikroorganismen soweit abgebaut und dementsprechend zerkleinert werden, bis sie das Filtrationsmodul passieren können.

Da die Faserstoffe als Besiedlungsfläche für die benötigten Mikroorganismen dienen, sind sie mit dem Großteil der Mikroorganismen vergesellschaftet. Zudem sorgen die Faserstoffe im Förderfilter dafür, dass eine ausreichend dichte Filtermatte entsteht, welche fein genug ist, um frei schwimmende Flocken aus Mikroorganismen und ggf. Pellets zurückzuhalten, die sich im Gärbehälter aus Mikroorganismen gebildet haben können. Somit werden die Mikroorganismen gleichermaßen zurückgehalten was die Stabilität der biologischen Prozesse verbessert, die im Gärbehälter ablaufen.

Ein Prozesshilfsstoff, der die Wirksamkeit der Filtration verbessert, kann vor dem Filter zugegeben werden. Beispielsweise kann ein Prozesshilfsstoff wie z. B. ein Flockungsmittel oder Flockungshilfsmittel zugegeben werden, um kleinere Partikel, die ansonsten filtergängig wären, entweder mit anderen Partikeln zu agglomerieren oder an die Flocken des Flockungsmittels zu binden, so dass diese Partikel im Ergebnis im Filter zurückgehalten werden können. Eine Dosiereinheit kann vorgesehen sein, um automatisch den Prozesshilfsstoff in einer gewünschten Dosierung dem Substrat zuzugeben, welches durch die Zuleitung zum Filter strömt. Die Einspeisestelle, an welcher der Prozesshilfsstoff in die Zuleitung gelangt, kann vorteilhaft möglichst nah am Gärbehälter angeordnet sein, so dass ein möglichst langer Abschnitt der Zuleitung als Reaktionsraum zur Verfügung steht, in dem der Prozesshilfsstoff über einen dementsprechend möglichst langen Zeitraum wirksam werden zu lassen, bevor das Substrat zusammen mit dem Prozesshilfsstoff in den Filter gelangt.

Grundsätzlich kann vorgesehen sein, dass die Zuleitung zur Vermeidung von Strömungsverlusten möglichst kurz bemessen ist. Die Untergrenze der Länge der Zuleitung ist einerseits durch den Abstand des Filters von dem Gärbehälter bestimmt und andererseits durch die Strömungsgeschwindigkeit des Substrats in der Zuleitung und dementsprechend durch die Zeitdauer, die der Prozesshilfsstoff zugunsten seiner Wirksamkeit erfordert, bevor das Substrat zusammen mit dem Prozesshilfsstoff in den Filter einströmt. Daher kann es vorgesehen sein, dass die Zuleitung bewusst länger ausgestaltet ist, als es für die rein strömungstechnische Verbindung zwischen Gärbehälter und Filter nötig wäre, um auf diese Weise die Länge des Reaktionsraums - und damit die zur Reaktion verfügbare Zeit für den Prozesshilfsstoff - zu verlängern. Beispielsweise kann die Zuleitung vom Gärbehälter zum Filter an Stelle eines geradlinigen einen um 90° gebogenen oder einen schlaufen- oder wendeiförmigen Verlauf aufweisen.

Durch das Zurückhalten der Faserstoffe steigt der Trockensubstanzgehalt im Gärbehälter. Zusammen mit den vermehrt vorhandenen Faserstoffen verschafft dies den Mikroorganismen eine zusätzliche Aufwuchsmöglichkeit, vergleichbar mit künstlich zugegeben Füllkörpern.

Alles zusammen führt dazu, dass der Biogasprozess durch Anwendung der Direktfiltration biologisch stabilisiert wird und das Substrat effizienter genutzt werden kann. Insbesondere können der Förderfilter sowie die Zu- und Rückleitung an den Gärbehälter in der Art anschließen, dass das Substrat bzw. die feste Phase in einem Kreislauf geführt und die dazu genutzte Anlage als ein geschlossenes System ausgestaltet ist, so dass der Eintritt von Sauerstoff in das System vermieden wird.

Ein Ausführungsbeispiel der Erfindung wird anhand der rein schematischen Darstellungen nachfolgend näher beschrieben; dabei zeigt
- Fig. 1: eine perspektivische Ansicht auf einen Wandabschnitt eines als Fermenter ausgestalteten Gärbehälters mit den daran anschließenden Komponenten für eine Direktfiltration,

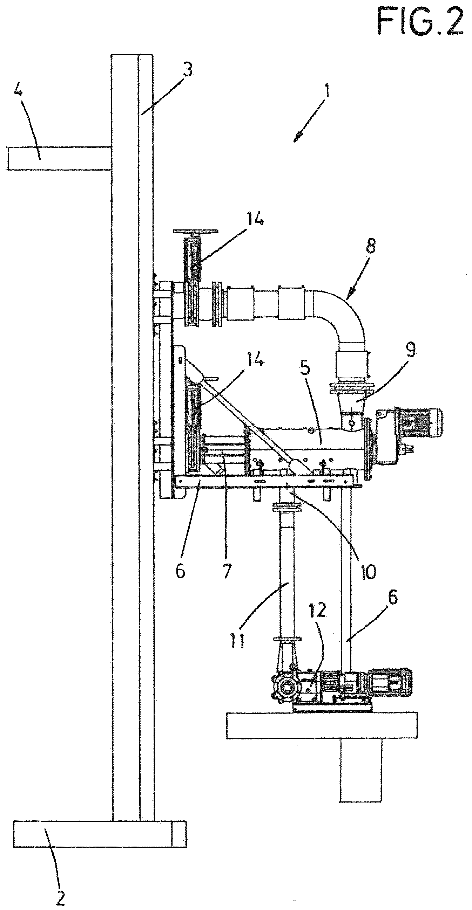

- Fig. 2: eine Seitenansicht auf die Situation von Fig. 1, und

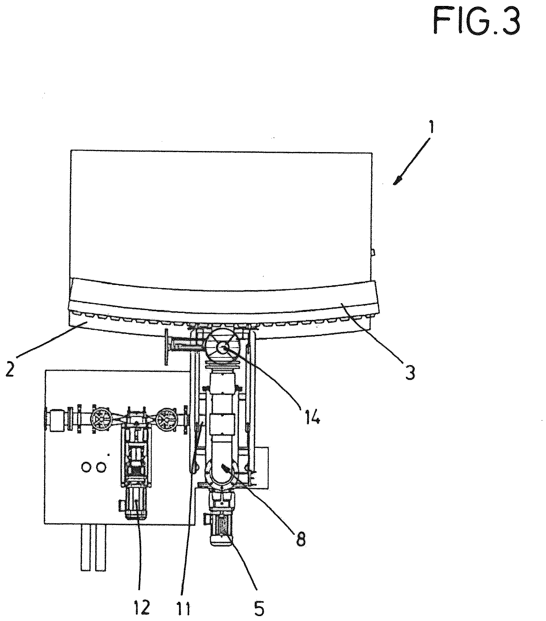

- Fig. 3: eine Draufsicht auf die Situation von Fig. 1.

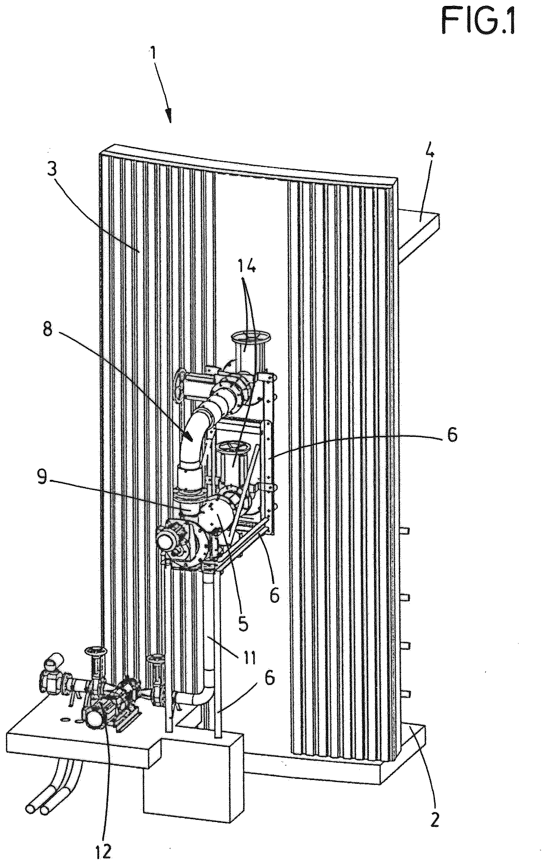

In den Zeichnungen ist ein Gärbehälter 1 lediglich ausschnittsweise dargestellt, wobei es sich bei dem dargestellten Ausführungsbeispiel des Gärbehälters 1 um einen Fermenter einer Biogasanlage handelt. Ausgehend von einer aus Beton bestehenden Bodenplatte 2 erstreckt sich eine insgesamt ringförmig verlaufende Wand 3 aufwärts. Eine in Fig 1 nicht eingezeichnete Decke schließt den Gärbehälter 1 nach oben ab. Als Oberkante des im Gärbehälter 1 befindlichen Substrats ist eine entsprechende Füllstandshöhe mit 4 angedeutet.

Ein Förderfilter 5 ist oberhalb des Bodenniveaus auf einer Konsole 6 außen vor der Wand 3 montiert und an die Wand 3 in der Art angeflanscht, dass ein Feststoffauslass 7 des Förderfilters 5 in die Wand 3 führt, so dass die feste Phase aus dem Förderfilter 5 durch eine Durchgangsöffnung bis in das Innere des Gärbehälters 1 gelangt. Ein Rohrstutzen, der die Wand 3 durchsetzt und bis zum Flansch des Feststoffauslasses 7 reicht, stellt eine besonders kurze Rückleitung dar, durch welche die feste Phase aus dem Förderfilter 5 in den Gärbehälter 1 strömt. Bei dem dargestellten Ausführungsbeispiel ist der Förderfilter 5 als Separator ausgestaltet.

Oberhalb des Förderfilters 5 verläuft ein Rohrbogen als Zuleitung 8 von der Wand 3 des Gärbehälters 1 bis zu einem Einlass 9 des Förderfilters 5. Auch hier ist ein Rohrstutzen in der Wand 3 vorgesehen, durch den das Substrat aus dem Gärbehälter 1 in die Zuleitung 8 gelangt. Allein durch die Wirkung der Schwerkraft strömt das Substrat aus dem Gärbehälter 1 in den Förderfilter 5.

Die Konsole 6 und dementsprechend auch der Förderfilter 5 ist in einer Höhe von mehr als 2 m oberhalb des Bodenniveaus des Gärbehälters 1 an dessen Wand 3 montiert. Die flüssige Phase, die den Förderfilter 5 durch einen Flüssigkeitsauslass 10 verlässt, strömt daher schwerkraftunterstützt durch eine Flüssigkeitsleitung 11 nach unten.

Je nach Ausgestaltung der Anlage, insbesondere des Separators, im vorliegenden Fall also des Förderfilters 5, kann der Separationsprozess beeinträchtigt sein, falls das Druckgefälle über den Filter nicht ausreicht, um die Flüssigkeit in dem benötigten Ausmaß durch den Separator zu leiten. Bei dem dargestellten Ausführungsbeispiel ist daher eine Förderpumpe 12 vorgesehen, die einerseits die gewünschte Separationsleistung sicherstellt und auch ermöglicht, die Flüssigkeit über eine größere Strecke fördern zu können.

Über Sperrventile 14 können sowohl die Zuleitung 8 als auch der Feststoffauslass des Förderfilters 5 vom Gärbehälter 1 strömungstechnisch abgetrennt werden.

### Bezugszeichen:

- 1: Gärbehälter
- 2: Bodenplatte
- 3: Wand
- 4: Füllstandshöhe
- 5: Förderfilter
- 6: Konsole
- 7: Feststoffauslass
- 8: Zuleitung
- 9: Einlass
- 10: Flüssigkeitsauslass
- 11: Flüssigkeitsleitung
- 12: Förderpumpe
- 14: Sperrventil

## Patentansprüche

1. Gärbehälteranordnung einer Biogasanlage,
mit einer Rohrleitung,
• die einen als Zuleitung (8) bezeichneten, von einem Gärbehälter (1) zu einem Einlass (9) eines Förderfilters (5) führenden Abschnitt,
wobei als Förderfilter (5) eine Filtrationseinrichtung bezeichnet ist, die ein oder mehrere bewegliche Elemente aufweist, die auf das zu filternde Medium eine aktive Förderwirkung ausüben,
• und einen als Rückleitung bezeichneten, von einem Feststoffauslass (7) des Förderfilters (5) zum Gärbehälter (1) führenden Abschnitt aufweist,
wobei die Zuleitung (8) dazu bestimmt ist, Substrat aus dem Gärbehälter (1) zu dem Förderfilter (5) zu führen, und wobei die Rückleitung dazu bestimmt ist, eine im Förderfilter (5) aus dem Substrat abgeschiedene feste Phase in den Gärbehälter (1) zurückzuführen,
**dadurch gekennzeichnet,**
**dass** die Zuleitung (8) vom Gärbehälter (1) abwärts zum Einlass (9) des Förderfilters (5) verläuft,
und **dass** der Förderfilter (5) an den Gärbehälter (1) in der Art angeflanscht ist, dass die feste Phase mittels einer Förderwirkung des Förderfilters (5) in den Gärbehälter (1) förderbar ist.

2. Gärbehälteranordnung nach Anspruch 1,
**dadurch gekennzeichnet,**
**dass** eine Flüssigkeitsleitung (11) von einem Flüssigkeitsauslass (10) des Förderfilters (5) dazu bestimmt ist, eine im Förderfilter (5) aus dem Substrat abgetrennte flüssige Phase aus dem Förderfilter (5) zu führen.

3. Gärbehälteranordnung nach Anspruch 1 oder 2,
**dadurch gekennzeichnet,**
**dass** eine Flüssigkeitsleitung (11) von einem Flüssigkeitsauslass (10) des Förderfilters (5) dazu bestimmt ist, eine im Förderfilter (5) aus dem Substrat abgetrennte flüssige Phase aus dem Förderfilter (5) zu führen,
wobei eine Förderpumpe (12) in die Flüssigkeitsleitung (11) geschaltet ist.

4. Gärbehälteranordnung nach einem der vorhergehenden Ansprüche,
**dadurch gekennzeichnet,**
**dass** die Zuleitung (8) als Rohrbogen ausgestaltet ist.

5. Gärbehälteranordnung nach einem der vorhergehenden Ansprüche,
**dadurch gekennzeichnet,**
**dass** die Zuleitung (8) und die Rückleitung frei von einer darin eingeschalteten Förderpumpe sind.

6. Gärbehälteranordnung nach einem der vorhergehenden Ansprüche,
**dadurch gekennzeichnet,**
**dass** der Gärbehälter (1) als Fermenter ausgestaltet ist.

7. Gärbehälteranordnung nach einem der vorhergehenden Ansprüche,
**dadurch gekennzeichnet,**
**dass** der Förderfilter (5) als Separator ausgestaltet ist.

8. Gärbehälteranordnung nach einem der vorhergehenden Ansprüche,
**dadurch gekennzeichnet,**
**dass** die Zuleitung (8) eine Einspeisestelle für die Zugabe eines Prozesshilfsstoffs aufweist.

9. Gärbehälteranordnung nach Anspruch 8,
**dadurch gekennzeichnet,**
**dass** die Einspeisestelle in einer möglichst großen Entfernung von dem Förderfilter (5) angeordnet ist.

10. Gärbehälteranordnung nach Anspruch 8 oder 9,
**dadurch gekennzeichnet,**
**dass** eine Dosiereinheit mit der Zuleitung (8) in der Art verbunden ist, dass der Prozesshilfsstoff mittels der Dosiereinheit in die Zuleitung (8) kontrolliert dosiert einbringbar ist.
